# EUROPEAN PATENT SPECIFICATION

(11) **EP 2 504 046 B1**
(45) Date of publication and mention of the grant of the patent: **04.10.2017**
(21) Application number: 10805296.0
(22) Date of filing: 22.11.2010
(51) Int. Cl.: A61M 5/00, A61M 5/145, A61M 5/148, A61M 5/168

(54) **DEVICE FOR DOSING AND ADJUSTING THE FLOW OF A RADIOPAQUE AGENT TO BE USED IN PERFORMING AN ANGIOGRAPHY**
VORRICHTUNG ZUR DOSIERUNG UND ANPASSUNG DES FLUSSES EINES RÖNTGENDICHTEN MITTELS ZUR DURCHFÜHRUNG EINER ANGIOGRAFIE
DISPOSITIF DE DOSAGE ET D'AJUSTEMENT DE L'ÉCOULEMENT D'UN AGENT RADIO-OPAQUE À UTILISER LORS DE LA RÉALISATION D'UNE ANGIOGRAPHIE

(30) Priority: 23.11.2009 IT BO20090763
(43) Date of publication of application: 03.10.2012
(73) Proprietor: Spark S.r.L., 40137 Bologna (IT)
(72) Inventor: ZANNOLI, Sebastiano, I-40137 Bologna (IT)
(74) Representative: Ruzzu, Giammario
(86) International application number: PCT/IB2010/002969
(87) International publication number: WO 2011/061614

(56) References cited:
- EP-A2- 0 446 715
- US-A- 4 976 162
- US-A1- 2009 093 734
- US-B1- 6 406 276

## Description

### BACKGROUND OF THE INVENTION

The present invention relates to the technical field concerning the production of apparatuses for diagnostic survey performed on human or animal body.

In particular, the present invention concerns a device aimed at supplying a predefined amount of a liquid or gaseous radiopaque agent, in an angiographic test.

### BRIEF DESCRIPTION OF THE PRIOR ART

It is known that an angiographic test includes injection of a radiopaque agent into a vascular cavity (artery or vein) and recording the radiological images of the area being examined. The diagnostic purpose of the angiography is to survey on and visualize the possible presence of structural anomalies of the vascular cavity being examined, including narrowings (stenosis) and dilatations (aneurisms) of the vessel lumen.

The technique is widely used with heart and vascular patients, due to the particularly high space resolution and diagnostic sensitivity that can be obtained.

The mainly used radiopaque agents are constituted by solutions containing liquid phase iodine, which, mixing with blood, allow obtaining of homogeneous and well defined images of the area being examined.

The use of the iodized radiopaque agent in the angiographic techniques is limited mainly by the subjective intolerance to the agent, first of all in the presence of some pathologic conditions (diabetic patients, nephropathic patients, etc.). Besides, the incidence of such pathologies is rapidly increasing as a consequence of the patients' increasing medium age.

Although the existence of such a limit is ascertained, the liquid phase radiopaque agent is used up to now all the same.

Carbon dioxide (CO₂) has been proposed as an alternative radiopaque agent, in order to overcome the disadvantages related to the angiographic techniques using the iodized agent. This gas is particularly suitable for this function since, once injected into the vessel, it allows to obtain an optimal radiographic visualization of the injection area and does not present the tolerance and disposal problems, as it is rapidly and totally exhaled by lungs.

The first injection devices used in the angiographic tests with CO₂ were of the manual type, and included simply the use of syringes filled with the gas and activated directly by the operator at the injection moment. With such syringes, the gas was injected directly in place, by means of a catheter, previously arranged in the area concerned. Such systems present the drawback deriving from the fact that they cannot ensure a complete and safe absence of air in the injection circuit, and they expose the patient to the risk of decompression sickness.

Afterwards, the injection devices, which have been initially prepared for use with liquid radiopaque agents, have been modified and adapted purposely, so as to make them suitable for the injection of the gaseous agent.

However, various proposed instrumental solutions, manual or automatic, leave unsolved a basic problem that influences the diagnostic result, that is that relating to the control of the gas infusion speed and to the preparation of the amount of gas to be injected. Actually, the simple adjustment of the forward movement speed of the injector plunger, as it occurs, on the other hand, with the injectors of liquid phase radiopaque agents, does not allow a satisfactory control of the injection flow, since the gas is naturally compressible. Moreover, also the injection pressure cannot be effectively controlled, since the volume variations caused in the injection syringe, due to the gas high compressibility and different draining speed, correspond to little predictable pressure variations, due to the variability of the particular injection conditions.

The obtained result is to achieve a non homogeneous and difficult to control opacification of the vascular cavity to be observed. This can make the diagnosis of possible pathologies difficult and sometimes little precise and reliable.

EP 0 446 715, in the name of E-Z-EM, Inc., discloses a contrast media injector, and specially a carbon dioxide injector, whose stated purpose is to enable the carbon dioxide to flow at varied flow rates, the varied flow rates correlated to the varied flow rate of blood in the animal's vascular system. The flow rate is adjusted by means of an electronic system (mechanism) that drives a valve during the course of an injection. This requires a precise feedback control of the injected gas pressure, which is obtained by means of suitable sensors placed along the injection conduit and connected to the electronic control system.

The injection device described above does not address the technical problem of precisely collecting a pre-defined amount of gas to be injected during an angiography before the injection is started, nor the one of injecting the collected gas at a substantially constant flow. Moreover, the whole system is complex, and it is subject to errors due to wrong calibration of some components, and to malfunctions due to component faults.

All of the above described devices are also rather complicated and expensive.

US 2009/0093734 A1 discloses an apparatus for the injection of carbon dioxide into a vascular structure.

US 6 406 276 B1 discloses a system for dispensing a fluid at relatively constant pressure with the use of a pre-tensioned resilient membrane.

### THE OBJECTS OF THE INVENTION

It is an object of the present invention to propose a device for dosing and administering carbon dioxide in an angiographic test, that allows a precise dosage thereof to be defined and a substantially constant injection flow to be obtained during the step of radiological survey.

Another object of the invention is to propose a device for dosing and administering carbon dioxide, that can be used in a sequential way without problems of contamination from the environment air.

A further object of the invention is to propose a device for dosing and administering carbon dioxide, that has a limited price and is highly reliable.

A still further object of the invention is to propose a device for dosing and administering a radiopaque agent in an angiographic test, that can be used with as much effectiveness for administering a liquid phase radiopaque agent.

### SUMMARY OF THE INVENTION

The above mentioned objects are wholly obtained in accordance with the contents of the claims and with preferred, but not exclusive embodiments of the invention, by a device for dosing and pressure adjustment of a radiopaque agent, in particular carbon dioxide, in performing angiography, supplied at a predefined pressure by a source of the same gas through a supply pipe. The device includes first dosing means, connected to the supply pipe and aimed at withdrawing measured amounts of carbon dioxide from the aforesaid source.

A first reservoir is connected to the first dosing means via a first flow diverter and contains a predefined amount of carbon dioxide to be supplied to an injection catheter.

The device includes also a second reservoir, connected to the alternative filling/draining means. The first and second reservoirs have a common yielding surface, in particular soft or moving, aimed at facilitating the gas pressure balance in the reservoirs. In addition, the second reservoir has an adjustable volume which is substantially larger than the volume of the first reservoir. An output pipe puts the first reservoir and the injection catheter in fluid communication, by means of a second flow diverter.

### BRIEF DESCRIPTION OF THE DRAWINGS

The characteristics of the invention, as they will appear from the claims, are pointed out in the following detailed description, with reference to the enclosed drawings, in which:
- Figure 1 is a schematic view of a device for supplying carbon dioxide, obtained in accordance with the invention;
- Figure 2 is a schematic view of the device of Figure 1 during a work step, in which the gas is charged;
- Figure 3 is the same view as the previous Figures, with the device during a work step, in which the injection catheter is washed;
- Figure 4 is the same view as the previous Figures, with the device during a work step, in which the radiopaque agent is injected;

### DETAILED DESCRIPTION OF PREFERRED EMBODIMENTS OF THE INVENTION

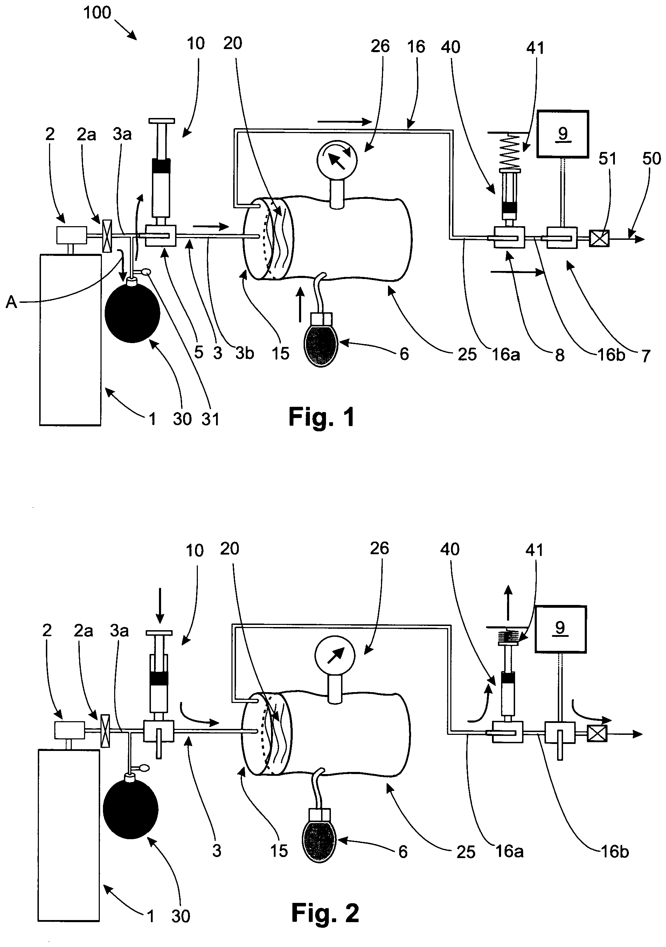

With reference to Figure 1 and to a preferred, but not exclusive embodiment of the invention, reference numeral 100 indicates, taken as a whole, a device for the dosage and adjustment of a radiopaque agent when performing angiographies, by means of a catheter 50, of known type, shown in the Figures wholly in schematic way, since its structure and its functions are not strictly relevant to the invention.

The following description, given by way of example, will make reference to the administration of a gaseous phase radiopaque agent, consisting of carbon dioxide. However, it is to be understood that the same inventive principle and the device that carries it out, can be used with as much advantage for administering a liquid phase radiopaque agent, though without the need to apply substantial changes to the device structure.

The device 100 is connected to a source 1 of carbon dioxide, having chemical purity adjusted in pressure, with the interposition of supplying means 2, and of a suitable filter 2a placed downstream thereof, by means of supply pipe 3. The source 1 can be formed either by a reservoir under pressure or by a fixed gas delivery system, depending on the logistic situation of the device 100 use. The filter 2a is of known type and is aimed at freeing the flow of carbon dioxide coming from the source 1 from the solid or other kind of impurities sporadically present therein.

An inlet of first flow stopping/diverter means 5, consisting of a three way diverter, whose functions will become evident from the following description, is connected to an upstream branch 3a of the supply pipe 3.

A flexible wall container 30, suitable for containing the gas necessary for An entire angiographic procedure (about 2 liters) at a pressure slightly higher than the atmospheric pressure, is connected upstream of the first diverter 5.

First dosing means 10, connected to the first diverter 5, are aimed at sucking from the container 30 and at containing a prefixed amount of carbon dioxide and at supplying it on command. In the above described Figures such first dosing means 10 are illustrated in the form of a syringe, which actually constitutes the simplest manual or motorized device for the functions to carry out. However, the illustration is not to be considered limitative and the syringe can be substituted by other types of gas dosing devices, possibly operated by suitable servomechanisms of known type.

Also the aforesaid first diverter 5 can be either of mechanical type or can be constituted by a group of electromagnetic valves, operated by suitable switches and aimed at interrupting or allowing the flow to the upstream branch 3a, to the downstream branch 3b of the supply pipe 3 and to the syringe 10 opening, respectively.

The device 100 includes also a first reservoir 15, connected to an outlet of the first diverter 5 by means of the downstream branch 3b of the supply pipe 3 and aimed at receiving a predefined amount of carbon dioxide from the first dispenser 10, so as to supply it, on command, to the aforesaid catheter 50 through the second flow stopping/diverting means 7, that will be described later on. The first reservoir 15 is preferably constituted by a soft, gas-proof bag, whose volume can be reduced with minimum force when it is empty.

The device 100 includes also a second reservoir 25, having capacity considerably bigger with respect to that of the first reservoir 15, and likewise constituted preferably by a soft bag, made of gas-proof material. For the invention purposes, it is preferred that the capacity of the second reservoir 25 is, as much as possible, bigger than that of the first reservoir 15 and is modifiable by, for example, introducing water therein.

The second reservoir 25 features, associated thereto, a device 6 for filling it with air at a predefined pressure and for subsequent draining it, and a manometer 26, aimed at measuring the pressure inside the reservoir 25. The filling device 6 is constituted for example, by a manually controlled or motorized pump, and a three way diverting valve, likewise manually or servo controlled.

The second reservoir 25 and the first reservoir 15 have a common yielding surface 20, obtained for example, by putting the two reservoirs one beside the other and fixing their contacting surfaces with glue or by another known technique. It is advisable to obtain a common surface 20 as big as possible, since it is aimed at facilitating the balance of the pressures inside the first reservoir 15 and the second reservoir 25.

According to a particular embodiment, not illustrated since immediately comprehensible, the first reservoir 15 and second reservoir 25 are housed inside a stiff, tubular structure, and their common surface 20 is constituted by a mobile wall inside the tubular structure, aimed at allowing a balance of the pressures inside the reservoirs.

According to another embodiment, likewise not illustrated for the same reasons, the first reservoir 15 is completely housed in the second reservoir 25. In this way, the common surface 20, which coincides with the entire casing of the first reservoir 15, is maximized.

In order to ensure that the maximum safety pressures inside the whole circuit are not exceeded, the container 30 and the second reservoir 25 are equipped with suitable safety valves, aimed at being opened at a predefined pressure, so as to make the exceeding gas go out.

A delivery duct 16, beginning from the first reservoir 15, has an upstream branch 16a that opens in an inlet of third flow stopping/diverting means 8. The latter are functionally constituted by a three way diverter, to which the output of the downstream portion 16b of the delivery duct 16 is connected.

A second dispenser device 40, connected to the third way of the third diverter 8, in its simplest form consists of a syringe, having smaller capacity with respect to the one that constitutes the first dispenser 10, aimed at receiving and delivering on command a predefined amount of carbon dioxide. However, it is understood that, in an automatic embodiment of the device 100, the second dispenser 40 can be likewise a different known type of gas dispenser, servo controlled or automatically operated. By way of example related to the last solution, in Figures from 1 to 4, a spring 41 is associated to the second syringe 40, acting on the piston of the syringe 40 with one of its ends, while the opposite end is kept fixed. This spring 41 is aimed at exerting an elastic reaction to the gas pressure inside the syringe 40, so as to allow it to be filled and drained in modes that will be described later on.

The above mentioned downstream portion 16b of the delivery duct 16 is connected to an inlet of the already mentioned second stopping/diverting means 7, which in turn consists of a three way diverter, whose outlet is connected to the catheter 50. On the other hand, the third way of the second diverter 7 is connected to a perfusion device 9 of known type, aimed at infusing a biocompatible liquid, for example normal saline solution, in the vessels to observe.

A pressure limiting device 31, connected to the previously described container 30, is aimed at preventing the pressure inside the container 30 from going beyond a prefixed safety value, for example, quantifiable in 100 mmHG.

The operation of the device 100 will be now described with reference to Figures from 2 to 4, which illustrate different work situations of the device during preparation and carrying out of an angiographic test.

Figure 1 instead illustrates a starting situation, in which some exam preparatory operations are carried out.

The first operation includes placing the first diverter 5 in a position in which it stops the flow from the supply pipe 3, and then opening the delivery device 2 of the source 1, so as to fill the container 30 with carbon dioxide at a predefined pressure through the filter 2a, as indicated with the arrow A.

This pressure is defined in such a way as to be slightly higher than the environment pressure, typically about 20 mmHg.

In this step, both the first syringe 10 and the second syringe 40 are empty and have their plungers completely inserted in the related cylinders. As far as the second syringe 40 in the illustrated embodiment is concerned, such position is maintained automatically by the elastic action of the spring 41.

With the catheter 50 still not in place in the blood vessel to be observed, the device draining is carried out, that is a flow of carbon dioxide is supplied to the first reservoir 15, to the output pipe 16 and to the catheter 50, until all the air possibly contained in the circuit is discharged. In this connection, the first diverter 5 is arranged so as to convey gas from the container 30 toward the downstream branch 3b of the supply pipe 3. The second diverter 7 and the third diverter 8 are placed in such a way as to put the output pipe 16 in communication with the catheter 50, and to exclude the perfusion device 9 from the circuit.

At this point, the catheter 50 is located in the observed place and the position of the second diverter 7 is modified, so as to interrupt the connection of the output pipe 16 and to connect the catheter 50 with the perfusion device 9. In this way, a preliminary perfusion of a flow of normal saline solution in the patient is carried out, so as to allow the correct positioning of the catheter in the vessel concerned.

Consequently, the air is introduced into the second reservoir 25, until a predefined injection pressure is reached, detectable by the manometer 26. This pressure depends on the particular clinical procedure adopted, and likewise, it is considerably higher with respect to the patient's endovascular pressure. The first dispenser 10 is filled with the desired amount of gas by means of the first diverter 5, sucking from the container 30, and is subsequently discharged, by its connection with the downstream branch 3b of the duct 3 by means of the first diverter 5. In this way, the amount of carbon dioxide contained therein is moved into the first reservoir 15. The pressure increase, proportional to the volume of the moved gas, is visualized by the manometer 26. During this step, also the second dispenser 40 is filled, after the third diverter 8 has been arranged in a way to put the upstream branch 16a of the delivery duct 16 in communication with the second dispenser 40, such that the pressure of the carbon dioxide present in the duct 16 overcomes the action of the spring 41. Figure 2 shows the above described work step.

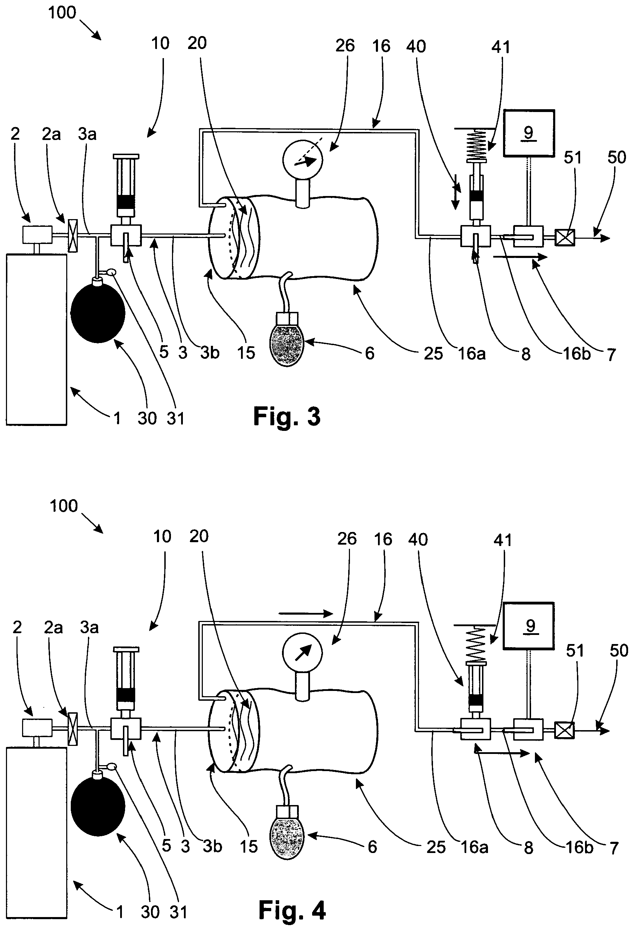

Consequently, the second diverter 7 interrupts the flow of the perfusion liquid from the device 9, and the third diverter 8 puts the second dispenser 40 in communication with the catheter 50 (Figure 3). Therefore, the elastic reaction force of the spring 41 discharges the carbon dioxide contained in the aforesaid dispenser 40, so as to inject it in the catheter and empty the liquid column present therein, with an operation called "washing" of the catheter 50.

Finally, the upstream part 16a of the output pipe 16 is put in communication with the downstream part 16b, acting on the third diverter 8 (Figure 4) and thus the carbon dioxide, contained in the first reservoir 15, is introduced into the catheter 50, and from it into the blood vessel to be observed, operating at the same time the radiological observation.

In this step, the flow of carbon dioxide introduced in the vessel is adjusted by the pressure present in the first reservoir 15. This pressure is kept substantially constant by the action of the second reservoir 25 on the first reservoir 15 through the common surface 20. The pressure inside the second reservoir 25 remains substantially constant, or in any case, it is subjected to a very moderate variation, even when the first reservoir 15 is emptied, since the volume of the latter is much smaller with respect to the second reservoir 25. The flow of carbon dioxide through the catheter will be then substantially constant during the whole angiographic test. On the other hand, also the amount of carbon dioxide that is injected is perfectly defined, since it corresponds exactly to the amount moved to the reservoir 15 by the dispenser 10.

Another flow stopping device 51 is provided downstream of the second diverter 7, aimed at stopping the flow in the catheter 50 on command, so as to assure the total closure of the pneumatic circuit toward the vessel, and consequently, the patient's total safety.

The above listed operations can be carried out manually by an operator, but they can be automated and controlled by a computerized system as well. Actually, the first dispenser 10 and the second dispenser 40 can be servo controlled, and the first diverter 5 and the second diverter 7, as well as the third diverter 8 can be constituted, for example, by a plurality of suitably arranged electromagnetic valves that can be operated by the aforesaid computerized system. Naturally, the diverters formed by a plurality of electromagnetic valves can be controlled manually as well, by means of switches situated in a control panel of the device.

As it has been already anticipated, the device 100 can be used also for administering a liquid phase radiopaque agent. In this case, the structure of the device 100 is substantially identical, with the first dispenser 10 that can be advantageously constituted by a peristaltic pump, aimed at acting on the supply pipe 3 to pump, on command, predefined doses of radiopaque agent to the first reservoir 15. The structure and the operation principle of a peristaltic pump are wholly known and can be applied immediately to the already described structure of the device 100.

The device 100 allows a series of advantages to be obtained with respect to the known devices, while performing an angiographic test with carbon dioxide. A first advantage derives from the possibility of defining a precise dosing of the carbon dioxide to be injected and of obtaining also a substantially constant injection flow.

Another advantage derives from the fact that the device 100 can be used more times without contamination problems, and it cannot be contaminated by the outside air during the exam, due to the pressure, which is always positive inside the entire circuit.

A further advantage of the device 100 is connected to its use with liquid phase radiopaque agents. These can be advantageously delivered at constant pressure, due to the presence of the second reservoir 25, in this way overcoming the disadvantages of the known injection devices. Indeed, in this way the subjectivity, and consequently uncertainty factor, connected to the operator's ability and strength, is eliminated, and the arising of overpressure, the injector locking and possible ducts breaking in case of the catheter obstruction or of the tip wedging in the closed vessels, is avoided.

A still further advantage of the device 100 derives from its reasonable price and extreme reliability of the entire injection system.

It is understood that what above has been described as a pure not limiting example. Therefore, possible modifications and variations of the invention are considered within the protective scope of the present technical solution, as described above and claimed below.

## Claims

1. A device for dosing and adjusting the flow of a radiopaque agent to be used while performing an angiography, said radiopaque agent being supplied at a predefined pressure by a source (1) through a supply pipe (3), said device (10) comprising: a first dosing syringe (10), connected to said source (1) and aimed at drawing, through said supply pipe (3), measured amounts of said radiopaque agent coming from said source (1); a first reservoir (15), selectively connected to said first dosing syringe (10) and aimed at receiving a predefined amount of said radiopaque agent from said first dosing syringe (10) in order to supply it to an injection catheter (50); an output pipe (16), aimed at selectively putting in fluid communication said first reservoir (15) with said injection catheter (50);
said device being **characterized by** further including:
a second reservoir (25), having an adjustable volume which is substantially larger than the volume of said first reservoir (15);
filling/draining means 6 connected to said second reservoir (25) aimed at supplying and draining air to and from said second reservoir (25), said first reservoir (15) and second reservoir (25) sharing at least one yielding surface (20), aimed at allowing the pressures in said reservoirs to balance.

2. A device according to Claim 1, **characterized in that** said reservoirs, first (15) and second (25), are entirely soft, and they consist of a pair of bags made of gas-proof material.

3. A device according to Claim 1, **characterized in that** said reservoirs, first (15) and second (25), are housed inside a stiff, tubular structure, said common surface (20) consisting of a mobile wall aimed at allowing the pressures inside said reservoirs to balance.

4. A device according to Claim 1 or Claim 4, **characterized in that** said first reservoir (15) is contained inside said second reservoir (25).

5. A device according to Claim 1, **characterized in that** a container (30) is provided upstream of said first dosing syringe (10), connected to said supply pipe (3) by means of first flow stopping/diverting means (5), aimed at receiving from said source (1) an amount of radiopaque agent, which is at least enough to perform an angiographic test and at storing it at a positive pressure.

6. A device according to Claim 1, **characterized in that** second dosing means (40) is provided upstream of said catheter (50), selectively connected to said output pipe (16), said second dosing means (40) being aimed at drawing a predefined amount of said radiopaque agent and at supplying it to said catheter (50) during a catheter (50) washing phase.

7. A device according to Claim 1, **characterized in that** said first dosing syringe (10) comprises a peristaltic pump, aimed at operating on said supply pipe (3) for pumping predefined doses of said radiopaque agent toward said first reservoir (15), under control of a device control unit.

## Patentansprüche

1. Eine Vorrichtung zum Dosieren und Einstellen der Strömung eines strahlungsundurchlässigen Mittels, das verwendet werden soll, während eine Angiographie durchgeführt wird, wobei das strahlenundurchlässige Mittel über eine Zufuhrleitung (3) mit einem vorgegebenen Druck durch eine Quelle (1) zugeführt wird, wobei die Vorrichtung (10) aufweist: eine erste Dosierungsspritze (10), die mit der Quelle (1) verbunden ist und darauf abzielt, durch die Zufuhrleitung (3) gemessene Mengen des von der Quelle (1) kommenden strahlenundurchlässigen Mittels zu ziehen;
ein erstes Reservoir (15), das selektiv mit der ersten Dosierspritze (10) verbunden ist und darauf abzielt, eine vordefinierte Menge des strahlenundurchlässigen Mittels von der ersten Dosierspritze (10) zu empfangen, um es einem Injektionskatheter (50) zuzuführen; ein Ausgangsrohr (16), das darauf abzielt, das erste Reservoir (15) selektiv mit dem Injektionskatheter (50) in Fluidverbindung zu bringen;
wobei die Vorrichtung **dadurch gekennzeichnet ist, dass** sie ferner aufweist:
ein zweites Reservoir (25) mit einem einstellbaren Volumen, das wesentlich größer ist als das Volumen des ersten Reservoirs (15);
eine mit dem zweiten Reservoir (25) verbundene Füll- / Entleerungseinrichtung 6, die darauf abzielt, Luft zu und von dem zweiten Reservoir (25) zuzuführen und abzulassen, wobei das erste Reservoir (15) und das zweite Reservoir (25) mindestens eine nachgiebige Oberfläche (20) teilen, die darauf abzielt, die Drücke in den Reservoirs auszugleichen.

2. Eine Vorrichtung nach Anspruch 1, **dadurch gekennzeichnet, dass** die Reservoirs, erste (15) und zweite (25), ganz weich sind, und sie aus einem Paar Beutel aus gasdichtem Material bestehen.

3. Eine Vorrichtung nach Anspruch 1, **dadurch gekennzeichnet, dass** die Reservoirs, erste (15) und zweite (25), innerhalb einer steifen, röhrenförmigen Struktur untergebracht werden, wobei die gemeinsame Oberfläche (20) aus einer beweglichen Wand besteht, die darauf abzielt, die Drücke in den Reservoirs auszugleichen.

4. Eine Vorrichtung nach Anspruch 1 oder Anspruch 4, **dadurch gekennzeichnet, dass** das erste Reservoir (15) innerhalb des zweiten Reservoirs (25) enthalten ist.

5. Eine Vorrichtung nach Anspruch 1, **dadurch gekennzeichnet, dass** vor der ersten Dosierspritze (10), ein Behälter (30), der mittels einer ersten Strömungsstopp- Umleiteinrichtung (5) verbunden ist, vorgesehen ist, der darauf abzielt, von der Quelle (1) eine Menge an strahlungsundurchlässigem Mittel, der mindestens genug ist, um einen angiographischen Test durchzuführen, zu empfangen und ihn bei einem positiven Druck zu speichern.

6. Eine Vorrichtung nach Anspruch 1, **dadurch gekennzeichnet, dass** eine zweite Dosiereinrichtung (40) stromaufwärts des Katheters (50) vorgesehen ist, wobei die zweite Dosiereinrichtung (40) darauf abzielt, eine vordefinierte Menge des strahlenundurchlässigen Mittels zu zeichnen und das Mittel dem Katheter (50) während einer Katheter (50) Waschphase zuzuführen.

7. Eine Vorrichtung nach Anspruch 1, **dadurch gekennzeichnet, dass** die erste Dosierungsspritze (10) eine peristaltische Pumpe aufweist, die darauf abzielt, auf der Zufuhrleitung (3) zu betätigen, zum Pumpen von vordefinierten Dosen des strahlungsundurchlässigen Mittels in Richtung des ersten Reservoirs (15), unter der Steuerung einer Vorrichtungssteuereinheit.

## Revendications

1. Dispositif pour doser et ajuster le flux d'un agent radio-opaque à utiliser lors de l'exécution d'une angiographie, ledit agent radio-opaque étant alimenté à une pression prédéfinie par une source (1) à travers un tuyau d'alimentation (3), ledit dispositif (10) comprenant: une première seringue doseuse (10), relié audite source (1) et destinée à prélever, à travers ledit tuyau d'alimentation (3), des quantités mesurées dudit agent radio-opaque provenant de ladite source (1);
un premier réservoir (15), connecté sélectivement à ladite première seringue de dosage (10) et destiné à recevoir une quantité prédéfinie dudit agent radio-opaque provenant de ladite première seringue de dosage (10) afin de la fournir à un cathéter d'injection (50); un tuyau de sortie (16), destiné à mettre sélectivement en communication de fluide ledit premier réservoir (15) avec ledit cathéter d'injection (50);
ledit dispositif étant **caractérisé en ce qu'**il comprend en outre:
un deuxième réservoir (25), ayant un volume réglable qui est sensiblement plus grand que le volume dudit premier réservoir (15);
des moyens de remplissage/vidange 6 connectés audit deuxième réservoir (25) destinés à fournir et à évacuer de l'air vers et depuis ledit deuxième réservoir (25), ledit premier réservoir (15) et deuxième réservoir (25) partageant au moins une surface meuble (20), afin de permettre l'équilibrage des pressions dans lesdits réservoirs.

2. Dispositif selon la revendication 1, **caractérisé en ce que** lesdits réservoirs, premier (15) et deuxième (25), sont entièrement mous, et ils se composent d'une paire de sacs en matériau anti-gaz.

3. Dispositif selon la revendication 1, **caractérisé en ce que** lesdits réservoirs, premier (15) et deuxième (25), sont logés à l'intérieur d'une structure tubulaire rigide, ladite surface commune (20) étant constituée d'une paroi mobile destinée à permettre l'équilibrage des pressions à l'intérieur desdits réservoirs.

4. Dispositif selon la Revendication 1 ou la Revendication 4, **caractérisé en ce que** ledit premier réservoir est contenu à l'intérieur dudit deuxième réservoir (25).

5. Dispositif selon la revendication 1, **caractérisé en ce que** un récipient (30) est prévu en amont de ladite première seringue de dosage (10), relié audit tuyau d'alimentation (3) au moyen de premiers moyens de d'interception/déviation (5), destiné à recevoir de ladite source (1) une quantité d'agent radio-opaque, qu'il est au moins suffisant pour effectuer un test angiographique, et à le stocker à une pression positive.

6. Dispositif selon la revendication 1, **caractérisé en ce que** deuxième moyen de dosage (40) sont prévu en amont dudit cathéter (50), et connecté sélectivement audit conduit de sortie (16), lesdites deuxième moyen de dosage (40) étant destinée à prélever une quantité prédéfinie dudit agent radio-opaque et à fournir l'agent au cathéter (50) pendant une phase de lavage du cathéter (50).

7. Dispositif selon la revendication 1, **caractérisé en ce que** ladite première seringue doseuse (10) comprend une pompe péristaltique, destinée à faire fonctionner ledit tuyau d'alimentation (3) pour pomper des doses prédéfinies de l'agent radio-opaque vers ledit premier réservoir (15), sous le contrôle d'une unité de commande de dispositif.
